(19) Europäisches Patentamt
European Patent Office
Office européen des brevets

(11) Publication number: **0 470 252 A1**

(12) **EUROPEAN PATENT APPLICATION published in accordance with Art. 158(3) EPC**

(21) Application number: **90906373.7**

(22) Date of filing: **27.04.90**

(86) International application number: **PCT/JP90/00571**

(87) International publication number: **WO 90/13542 (15.11.90 90/26)**

(51) Int. Cl.5: **C07D 215/56**, C07D 471/04, C07D 489/06, C07D 513/06, A61K 31/435, A61K 31/47, A61K 31/495, A61K 31/535, A61K 31/54

(30) Priority: **28.04.89 JP 110800/89**
**24.07.89 JP 191062/89**
**23.02.90 JP 429/89**

(43) Date of publication of application: **12.02.92 Bulletin 92/07**

(84) Designated Contracting States: **AT BE CH DE DK ES FR GB IT LI LU NL SE**

(71) Applicant: **DAIICHI PHARMACEUTICAL CO., LTD.**
**14-10, Nihonbashi 3-chome**
**Chuo-ku, Tokyo 103(JP)**

(72) Inventor: **OHTA, G Daiichi Pharm. Co., Ltd. Res. Inst.**
**16-13, Kitakasai 1-chome Edogawa-ku**
**Tokyo 134(JP)**
Inventor: **FURUSAWA, M Daiichi Pharm. Co., Ltd. Res. Inst.**
**16-13, Kitakasai 1-chome Edogawa-ku**
**Tokyo 134(JP)**
Inventor: **NOZAKI, Junko**
**15-6-306, Shinjuku 6-chome Shinjuku-ku**
**Tokyo 160(JP)**
Inventor: **SATO, Y Daiichi Pharm. Co., Ltd. Res. Inst.**
**16-13, Kitakasai 1-chome Edogawa-ku**
**Tokyo 134(JP)**
Inventor: **IINO, T Daiichi Pharm. Co., Ltd. Res. Inst.**
**16-13, Kitakasai 1-chome Edogawa-ku**
**Tokyo 134(JP)**

(74) Representative: **Kinzebach, Werner, Dr. et al**
**Patentanwälte Reitstötter, Kinzebach und Partner Sternwartstrasse 4 Postfach 86 06 49**
**W-8000 München 86(DE)**

(54) **ANTI-HIV DRUG.**

(57) Fluorinated pyridonecarboxylic acid derivatives such as ofloxacin, levofloxacin, norfloxacin, enoxacin, ciprofloxacin, lomefloxacin, fleroxacin, difloxacin and tosufloxacin have an anti-HIV activity, so that they are useful drugs for treating diseases caused by HIV. The anti-HIV activities of these derivatives can be enhanced when they are used together with azidothymidine, dideoxyinosine or dideoxycytidine, so that they are promising also in this respect.

EP 0 470 252 A1

## TECHNICAL FIELD

This invention relates to an anti-HIV agent.

## BACKGROUND ART

It is known that human immunodeficiency virus (HIV) is causative of acquired immunodeficiency syndrome, the so-called AIDS, in humans infected therewith. As a result of investigations in search of anti-HIV agents, several effective agents have already been developed. Neither of them is fully satisfactory, however. Thus, even azidothymidine (AZT), which is said to be an effective chemotherapeutic agent, cannot be free from problems; long-term administration of AZT will cause adverse reactions due to bone marrow suppression, such as anemia and neutropenia, and allow development of resistance to AZT in HIV. It has now been found that various fluorine-containing pyridonecarboxylic acid derivatives can inhibit producing progeny of HIV in cells infected therewith on one hand and, on the other, do not inhibit host cell proliferation and further that when used in combination with azidothymidine, dideoxyinosine or dideoxycytidine, they can produce good effects as well. The present invention is based on these findings.

## DISCLOSURE OF THE INVENTION

This invention relates to an anti-human immunodeficiency virus agent which comprises a fluorine-containing pyridonecarboxylic acid derivative as an active ingredient and further to a drug composition which comprises a fluorine-containing pyridonecarboxylic acid derivative as an active ingredient in combination with azidothymidine, dideoxycytidine (2',3'-dideoxycytidine) (J. E. Dahlberg et al., 1987, Proc. Natl. Acad. Sci. USA, 84: 2469-2473) or dideoxyinosine (2',3'-dideoxyinosine) (R. Yarchoan et al., 1989, Science 245: 412-145) for incresing the anti-HIV effect.

The fluorine-containing pyridonecarboxylic acid derivative can be represented by the formula:

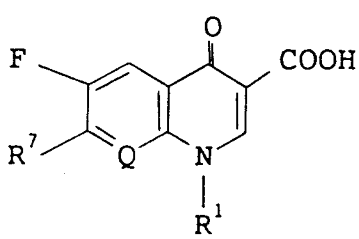

wherein Q is =N- or =C($R^8$)- in which $R^8$ is H, F, Cl, lower alkyl or lower alkoxyl, or when taken together with $R^1$, -$OCH_2CH(R^{81})$-, -$SCH_2CH(R^{81})$- or -$OCH_2N(R^{81})$- to complete an additional ring, $R^{81}$ being H or lower alkyl; $R^1$ is lower alkyl, cyclopropyl, halocyclopropyl (in particular, cis-fluorocyclopropyl), haloethyl, vinyl, phenyl or halophenyl (in particular, 2-fluorophenyl, 4-fluorophenyl or 2,4-difluorophenyl); $R^7$ is a nitrogen-containing heterocyclic group (e.g. piperazinyl, pyrrolidinyl or piperidinyl), which may optionally be substituted by one or more substituents, typically a group of the formula

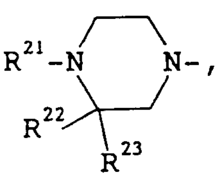, 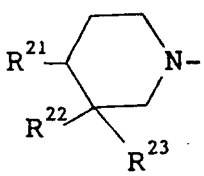 or 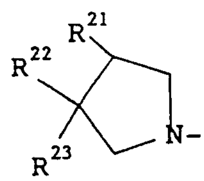

wherein $R^{21}$, $R^{22}$ and $R^{23}$ each independently is hydrogen atom, halogen atom, amino, lower alkyl, lower alkoxyl or amino-lower alkyl and two of them may be combined with each other to form a spiro ring. As specific examples of the nitrogen-containing heterocyclic group, there may be mentioned, among others, 3-aminopyrrolidinyl, 3-methylaminopyrrolidinyl, 3-dimethylaminopyrrolidinyl, 3-ethylaminopyrrolidinyl, 3-propylaminopyrrolidinyl, 3-isopropylaminopyrrolidinyl, 3-amino-4-methylpyrrolidinyl, 3-amino-5-methylpyr-

rolidinyl, 3-amino-4,5-dimethylpyrrolidinyl, 3-methylamino-4-methylpyrrolidinyl, 3-methylamino-5-methylpyrrolidinyl, 3-methylamino-4,5-dimethylpyrrolidinyl, 3-dimethylamino-4-methylpyrrolidinyl, 3-dimethylamino-5-methylpyrrolidinyl, 3-dimethylamino-4,5-dimethylpyrrolidinyl, 3-methylpiperazinyl, 4-methylpiperazinyl, 3,4-dimethylpiperazinyl, 3,5-dimethylpiperazinyl, 3,4,5-trimethylpiperazinyl, 4-ethyl-3,5-dimethylpiperazinyl, 4-isopropyl-3,5-dimethylpiperazinyl, 3-aminomethylpyrrolidinyl, 3-methylaminomethylpyrrolidinyl, 3-(1-amino)-ethylpyrrolidinyl, 3-(1-methylamino)ethylpyrrolidinyl, 3-(1-ethylamino)ethylpyrrolidinyl, 3-(1-amino)-propylpyrrolidinyl, 3-(1-methylamino)propylpyrrolidinyl, 3-aminopyrrolidinyl, 3-amino-4,4-dimethylpyrrolidinyl, 7-amino-5-azaspiro[2.4]heptan-5-yl, 8-amino-6-azaspiro[3.4]-octan-6-yl, 1,4-diazabicyclo[3.2.1]-octan-4-yl, 3,8-diazabicyclo[3.2.1]octan-3-yl, 8-methyl-3,8-diazabicyclo[3.2.1]octan-3-yl, 8-ethyl-3,8-diazabicyclo[3.2.1]octan-3-yl and the like. These quinolinecarboxylic acid compounds, naphthyridinecarboxylic acid compounds and pyridobenzoxazinecarboxylic acid compounds are disclosed, for example, in JP-A-53-141286, JP-A-58-74667, JP-A-59-67279, JP-A-60-64979, JP-A-60-174786, JP-A-60-228469 (The term "JP-A" used herein means "an unexamined published Japanese Patent Application") and in Japanese Patent Applications Nos. 1-106762, 1-123366, 1-156316 and 1-223910 and can be produced by the methods disclosed in the respective patent specifications. Among such compounds, those fluorine-containing pyridonecarboxylic acid derivatives which have so far been developed and commercialized are known under the designation of new quinolones to have excellent antimicrobial activity [cf. Naika (Internal Medicine), 62 (1), 28-33 (1988) and Farumashia, 25 (5), 434-440]. Antimicrobial compounds of the same type which are to be developed in the future, namely further compounds of the above formula, which have the 6-fluoroquinoline-3-carboxylic acid, 6-fluoro-1,8-naphthyridine-3-carboxylic acid or 9-fluoro-2,3-dihydro-7-oxo-7H-pyrido[1,2,3-de][1,4]benzoxazine-6-carboxylic acid skeleton with one or more appropriate substituents, will show excellent anti-HIV activity as well when used either alone or in combination with azidothymidine, deoxyinosine or deoxycytidine. In particular, ofloxacin and S(-)-9-fluoro-2,3-dihydro-3-methyl-10-(4-methyl-1-piperazinyl)-7-oxo-7H-pyrido[1,2,3-de][1,4]benzoxazine-6-carboxylic acid hemihydrate (levofloxacin; DR-3355) have a marked anti-HIV effect. Furthermore, ofloxacin (or levofloxacin), when used in combination with azidothymidine (or dideoxycytidine or dideoxyinosine), produces a marked anti-HIV effect as well. Those which are on the market and can be used in the practice of the invention include ofloxacin [OFLX; (±)-9-fluoro-3-methyl-10-(4-methyl-1-piperazinyl)-7-oxo-2,3-dihydro-7H-pyrido[1,2,3-de][1,4]-benzoxazine-6-carboxylic acid], norfloxacin [NFLX; 1-ethyl-6-fluoro-1,4-dihydro-4-oxo-7-(1-piperazinyl)-3-quinolinecarboxylic acid], ciprofloxacin hydrochloride [CPFX; 1-cyclopropyl-6-fluoro-1,4-dihydro-4-oxo-7-(1-piperazinyl)-3-quinolinecarboxylic acid], enoxacin [ENX; 1-ethyl-6-fluoro-1,4-dihydro-4-oxo-7-(1-piperazinyl)-1,8-naphthyridine-3-carboxylic acid], tosufloxacin [TFLX; (±)-7-(3-amino-1-pyrrolidinyl)-6-fluoro-1-(2,4-difluorophenyl)-1,4-dihydro-4-oxo-1,8-naphth yridine-3-carboxylic acid p-toluenesulfonate monohydrate] and lomefloxacin [LFLX; (±)-1-ethyl-6,8-difluoro-1,4-dihydro-7-(3-methyl-1-piperazinyl)-4-oxo-3-quinolinecarboxylic acid hydrochloride], among others. Such compounds may also be used in the form of salts, hydrates or the like. The anti-HIV composition according to the present invention can be administered by such conventional methods as oral administration and intravenous drip. For oral administration, said composition may have the form of tablets, capsules, granules, powders, syrups and the like. For intravenous drip, said composition may have the form of solutions prepared by adding a solubilizing agent, such as an acid or alkali, as necessary. For oral administration, a suitable daily dose of the fluorine-containing pyridonecarboxylic acid for adult humans lies within the range of 100 to 1,000 mg, in particular 200 to 600 mg. In the case of levofloxacin, a dose of 150 to 300 mg (for oral administration) is generally suitable. The dose of azidothymidine for oral administration is suitably 600 to 1,800 mg, in particular 1,200 mg. The doses can be adjusted depending on the patient's age and symptom. In particular, when ofloxacin or the like fluorine-containing pyridonecarboxylic acid derivative is used in combination with azidothymidine, dideoxycytidine or dideoxyinosine, both the active agents may be administered either simultaneously or separately at certain time intervals. The numbers of administrations of the two agents may not be always the same and it is suitable to administer azidothymidine in 4 to 6 divided doses in a day and the fluorine-containing pyridonecarboxylic acid derivative in 2 to 4 divided doses in a day. The following examples are further illustrative of the invention. Human T cell-derived, T4 antigen-positive CEM cells (gift of Institut Pasteur, France) were used as host cells for HIV. Cell culture was carried out in a carbon dioxide-containing atmosphere in an incubator maintained at 37°C according to a conventional method [cf. National Institute of Health (Japan) Alumni Association (ed.): "Virus Jikkengaku Soron (Experiments in Virology)", pages 131-151]. RPMI 1640 medium (Gibco) containing 10% fetal calf serum (FCS) was used as the cell culture medium. The culture supernatant ($5 \times 10^6$ $TCID_{50}$/ml) obtained by subjecting CEM cells to HIV-1 (LAV 1 strain) infection and stored at -70°C was used as the HIV source. The abbreviation "$TCID_{50}$" means "50% tissue culture infectious dose" (cf. Hisao Uetake (ed.): "Virus-gaku (Virology)", Rikogakusha, page 472) and "MOI" means "multiplicity of infection" (cf. the monograph cited just above, page 36).

## BEST MODES FOR CARRYING OUT THE INVENTION

### Example 1: Cytotoxicity

The cell culture medium (10 ml) placed in a plastic culture flask (25 $cm^2$) was inoculated with CEM cells to a cell density of 2.5 x $10^5$ cells/ml and incubation was performed for 3 days. Then, the culture was transferred to a 15-ml plastic centrifuge tube and centrifuged at 1,000 revolutions per minute for 5 minutes for medium removal. A fresh portion of the medium was added to the cells separated, the cell density being adjusted to 5.0 x $10^5$ cells/ml. A total volume of 1.0 ml of a composition consisting of 0.5 ml of the cell suspension (5.0 x $10^5$ cells/ml) prepared in the above manner, 0.4 ml of the medium (RPMI 1640, 10% FCS) and 0.1 ml of a physiological saline solution of the test substance with a concentration 10 times the final concentration was incubated on a 24-well plastic culture plate for 4 days. The cells in each culture plate well were then suspended uniformly in the medium by pipetting and a 0.5 ml portion of the suspension was separated. A fresh 2 ml portion of the test substance-free medium was added to the remaining cell suspension (0.5 ml) and, after further 3-day incubation, a cell suspension was produced in the same manner and a portion thereof was collected and subjected to viable cell count and viable cell percentage determinations by trypan blue staining. As a result, ofloxacin, ciprofloxacin, enoxacin, norfloxacin, levofloxacin and azidothymidine each showed no cytotoxicity at concentrations not exceeding 10 µg/ml. Two repetitions of the same test gave the same results.

### Example 2: Anti-HIV activity

The medium for cell culture (10 ml) placed in a plastic culture flask (25 $cm^2$) was inoculated with CEM cells at a cell density of 2.5 x $10^5$ cells/ml. After 3 days of incubation, the culture was transferred to a 15-ml plastic centrifuge tube and subjected to centrifugation at 1,000 revolutions per minute for 5 minutes for sedimentation of cells and removal of the culture medium.

The stock viral suspension was added to the host cells thus prepared to MOI = 0.05 and the mixture was maintained at 37°C for 90 minutes with occasional stirring at 30-minute intervals for viral adsorption. The medium was added to these HIV-infected cells to adjust the cell density to 5.0 x $10^5$ cells/ml. The resultant HIV-infected cell suspension was used as an inoculum cell suspension.

A mixture (1.0 ml) of 0.5 ml of the HIV-infected cell suspension, 0.4 ml of the medium (10% FCS in RPMI 1640) and 0.1 ml of a physiological saline solution of the test compound at a concentration 10 times the final concentration was placed in each well of a 24-well plastic culture plate. The test substance final concentration was adjusted to 10 µg/ml, 5 µg/ml, 2.5 µg/ml or 1 µg/ml. The "no addition" wells (0 µg/ml) served as controls.

After 4 days of incubation following viral infection, the cells in each well on the 24-well culture plate were suspended uniformly by pipetting and a 0.5 ml portion of the suspension was separated. A 2.0 ml portion of the test substance-free fresh medium was added to the remaining 0.5 ml portion of the cell suspension, followed by incubation. (For ofloxacin and levofloxacin, continued (repeated) addition experiments were also performed.)

Seven days after viral infection, pipetting was performed to give a uniform cell suspension in each well and a 0.5 ml portion of the suspension was separated.

Incubation was continued and, 10 days after viral infection, a uniform cell suspension was produced in the same manner in each well and a 1.5 ml (or 2.0 ml) portion thereof was separated. A 2.0 ml portion of the fresh medium free of (or containing) the test substance was added to the remaining 0.5 ml portion of the cell suspension, followed by continued incubation.

Fifteen days after viral infection, the cells in each well were suspended uniformly in the medium in the same manner as above and a 2.0 ml portion of the suspension was separated. A 2.0 ml portion of the fresh medium free of (or containing) the test substance was added to 0.5 ml of the remaining cell suspension and incubation was continued. Medium exchange was also performed in the same manner 25 days after viral infection.

The cell suspensions collected at the day shown in each of Tables 1 to 8 were tested for viable cell count and viable cell percentage by trypan blue staining. At the same time, viral antigen-positive cell percentage determination was carried out by the immunofluorescence method using an anti-HIV antiserum isolated from a healthy carrier of HIV. The results thus obtained are shown below in Tables 1 to 8. The numerical values given each is the mean for the respective two wells on the culture plate. The viable cell count is the total cell count minus the count of dead cells stained with trypan blue. The viable cell percentage is the percentage of viable cells among all the cells including dead cells. The viral antigen-positive cell percentage is the percentage of cells indicating antigen expression as found among all the cells including dead cells.

In the control experiments without treatment with any compound, the cell count decreased after 17 days and all the cells were dead after 30 days, as shown in Table 1; the viral antigen test always gave a positive result. Treatment with ofloxacin or levofloxacin contributed to the maintenance of cell proliferation and suppressed the viral antigen-positive percentage. Azidothymidine treatment, as shown in Table 3, resulted in temporary maintenance of cell proliferation at high concentrations, which was, however, followed by decreases in cell count and by proliferation of the virus as evidenced by the increase in viral antigen-positive percentage. In view of the thus-obtained favorable results, the number of test compounds was increased and time course examination was conducted in a more detailed manner (Tables 4 to 8). The fluorine-containing pyridonecarboxylic acid derivatives given in Tables 4 to 8 were effective against the cytopathic effect of the virus and showed viral proliferation inhibiting activity, with the same tendency as shown by levofloxacin and ofloxacin (Tables 1 and 2), although the degree of antiviral activity varied depending on the compound.

Example 3:

(i) Method of testing the combination of a fluorine-containing pyridonecarboxylic acid derivative (levofloxacin) and azidothymidine for anti-HIV activity

The inoculum cell suspension prepared in Example 1 was adjusted to a cell density of $2.5 \times 10^5$ cells/ml by adding the fresh medium for cell culture. The cell suspension was inoculated, in 1 ml portions into wells of a 24-well culture plate. A physiological saline solution of the test compound(s) azidothymidine and/or levofloxacin at a concentration 100 times the final concentration was added, in 10-µl portions, to wells of the 24-well culture plate. The virus-infected cells were then cultured at 37°C for 4 days in a $CO_2$ incubator.

The cells in each well of the 24-well culture plate were suspended uniformly by pipetting, a 0.5 ml portion of the uniform cell suspension was taken out into a disposable tube for viable cell counting and HIV antigen detection.

A 2 ml portion of the fresh medium free of (or containing) the test compound was added to the remaining cell suspension (0.5 ml) and incubation was continued.

Seven days after viral infection, the cells in each well of the culture plate were suspended uniformly by pipetting and a 0.5 ml portion of the cell suspension was taken out.

Ten days after viral infection, the cells in each well of the culture plate were suspended uniformly by pipetting and 1.5 ml portion of the cell suspension was separated. A 2 ml portion of the medium free of (or containing) the test compound was added to the remaining cell suspension (0.5 ml) and incubation was continued.

Fifteen days after viral infection, the cells in each well of the culture plate were suspended uniformly by pipetting and a 2 ml portion of the cell suspension was taken out. A 2 ml portion of the medium free of (or containing) the test compound was added to the remaining cell suspension (0.5 ml) and incubation was continued.

Medium exchange was further conducted in the same manner 20 days and 25 days after viral infection.

The cell suspension portions separated 4 days, 7 days, 10 days, 15 days, 20 days and 30 days following viral infection were subjected to viable cell counting and viable cell percentage determination using the trypan blue staining technique. At the same time, viral antigen-positive cell percentage determination was performed by the immunofluorescence method using an anti-HIV serum isolated from a healthy carrier of HIV. The results obtained are shown in Table 9. The numerical values shown each is the mean of two wells.

(ii) Effects of the combined use of levofloxacin and azidothymidine

When CEM cells were infected with an HIV dilution ($TCID_{50}$: $5 \times 10^6$/ml; MOI: 0.05), 50% to 91% of the cells turned viral antigen-positive in 4 days, rapid decreased in cell count were observed on day 7 and thereafter, and all the cells were dead on day 20 (Table 9).

HIV-infected CEM cells were treated with an azidothymidine solution at a final concentration of 0.1 to 1.0 µg/ml for 4 days and incubated for 30 days with medium exchanging at regular intervals as mentioned above. Rapid decreases in cell count due to HIV infection were observed on day 15 of incubation, with a delay of about 8 days as compared with the untreated control (day 7). However, even the cells treated with

azidothymidine were found all dead on day 20. Continuous treatment with the same agent showed the same tendency.

Another culture plate was inoculated with HIV-infected CEM cells and, after addition of a levofloxacin solution to a final concentration of 1 µg/ml or 10 µg/ml, levofloxacin treatment was performed for 4 days or continuously for 30 days. In this culture system, a temporary decrease in cell count from day 7 to day 15 was followed by reproliferation, whereby cells survived cell damage by HIV and the cell count was maintained at a high-density level (Table 9).

Separately, a further culture plate was inoculated with HIV-infected CEM cells, which were then treated with a solution containing the two test substances levofloxacin (final concentration: 1.0 or 10 µg/ml) and azidothymidine (final concentration: 0.1 or 1.0 µg/ml). In this combined treatment culture system, the degree of decrease in cell count was remarkably low on day 4 to day 10 as compared with the no-treatment control and almost all cells survived. In this case, such rapid decrease in cell count following delayed infection as seen in the case of single treatment with azidothymidine was not observed. The combined use reduced the extent of such temporary decrease in cell count on day 7 to day 15 of incubation as seen in the single treatment with levofloxacin. In such respect, the combined use of both compounds produced beneficial effects.

Furthermore, testing for viral antigen detection revealed that cells treated with azidothymidine alone all turned positive eventually and died, but that cells subjected to the combined treatment, like cells treaded with levofloxacin alone, gave a decreased positive rate as low as 1% or below in the later part of the incubation period, with no antigen detected, and most of them survived as virus-free cells. In the case of continued treatment, too, substantially the same results were obtained.

Example 4: Dosage form example (capsules)

| | |
|---|---|
| Fluorine-containing pyridone-carboxylic acid (e.g. ofloxacin or levofloxacin) | 100.0 mg |
| Corn starch | 23.0 mg |
| CMC calcium | 22.5 mg |
| Hydroxypropylmethylcellulose | 3.0 mg |
| Magnesium stearate | 1.5 mg |
| Total | 150.0 mg |

Example 5: Dosage form example (injectable solution)

| | |
|---|---|
| Levofloxacin | 20 g |
| Sodium chloride | 6 g |
| Distilled water for injection to make | 1,000 ml |

INDUSTRIAL APPLICABILITY

(i) The fluorine-containing pyridonecarboxylic acid derivatives according to the present invention exhibit antiviral activity, though slowly, reducing the viral antigen-positive cell percentage with time and inhibiting virus repropagation. Such substances as azidothymidine, dideoxyinosine and dideoxycytidine initially inhibit the propagation of HIV but later allow repropagation of the virus with time, which leads to cell damage. The fluorine-containing pyridonecarboxylic acid derivatives show anti-HIV activity by other mechanisms of action than those of azidothymidine, dideoxyinosine or dideoxycytidine and have properties promising their use as anti-HIV agents.

(ii) When any of the fluorine-containing pyridonecarboxylic acid derivatives and azidothymidine, dideoxycytidine or dideoxyinosine are used combinedly, the cell life prolonging effect of the fluorine-containing pyridonecarboxylic acid derivative as well as the infection delaying effect of azidothymidine (or dideoxyinosine or dideoxycytidine) is produced additively. In the combined use of both compounds, the viral antigen-positive cell percentage is reduced with time and any increase in viral antigen is not observed even thereafter. Treatment with azidothymidine (or dideoxyinosine or dideoxycytidine) alone initially

inhibits the propagation of HIV but later allows HIV to propagate and cells to become viral antigen-positive. Therefore, the combined use of the fluorine-containing pyridonecarboxylic acid and azidothymidine (or dideoxycytidine or dideoxyinosine) can be expected to produce better effects as compared with single treatment with either compound. The fluorine-containing pyridonecarboxylic acid derivatives presumably show their anti-HIV activity by other mechanisms of action than those of azidothymidine (or dideoxycytidine or dideoxyinosine) and the combined use of both agents may be said to be a favorable method for the treatment of HIV infection.

As for the toxicity of the compounds to be used in accordance with the invention, the following $LD_{50}$ values as determined in mice may be mentioned, for instance; 244 mg/kg (intravenous) for levofloxacin, 5,450 mg/kg (oral) for ofloxacin, and not less than 4,000 mg/kg (oral) for ciprofloxacin, enoxacin and norfloxacin.

Table 1

Efficacy of Ofloxacin on Cytophatic Effect in HIV-1-Infected CEM cells

| Test Compound (μg/ml) | Viable Cell Count ($x10^4$ cells/ml) | | Viable Cell Percentage (%) | | Viral Antigen-Positive Cell Percentage (%) | |
|---|---|---|---|---|---|---|
| | After 17 Days | After 30 Days | After 17 Days | After 30 Days | After 17 Days | After 30 Days |
| 10 | 170 | 260 | 88 | 94 | 23 | 4 |
| 1 | 10 | 25 | 38 | 27 | NT | 67 |
| 0 (control) | 8 | 0 | 25 | 0 | 90 | ≥90 |

Note: Cell count at the time of inoculation: $2.5 \times 10^5$ cells/ml.

Viable cell count: Total cell count minus count of dead cells stained with trypan blue.

Viable cell percentage: Percentage of viable cells among all cells including dead cells.

Viral antigen-positive cell percentage: Percentage of cells indicatingantigen expression as found among all cells including dead cells.

Table 2

Efficacy of Levofloxacin on Cytophatic Effect in HIV-1-Infected CEM Cells

| Test Compound (μg/ml) | Viable Cell Count ($x10^4$ cells/ml) After 12 Days | Viable Cell Count ($x10^4$ cells/ml) After 25 Days | Viable Cell Percentage (%) After 12 Days | Viable Cell Percentage (%) After 25 Days | Viral Antigen-Positive Cell Percentage (%) After 12 Days | Viral Antigen-Positive Cell Percentage (%) After 25 Days |
|---|---|---|---|---|---|---|
| 10 | 100 | 380 | 74 | 97 | 34 | 6 |
| 1 | 96 | 79 | 57 | 53 | 55 | 26 |
| 0 (control) | 27 | 5 | 35 | 36 | 90 | 90 |

Note: Cell count at the time of inoculation: $2.5 \times 10^5$ cells/ml. For explanation of terms, see Table 1.

Table 3

Efficacy of Azidothymidine on Cytophatic Effect in HIV-1-Infected CEM Cells

| Test Compound (μg/ml) | Viable Cell Count ($\times 10^4$ cells/ml) | | | Viable Cell Percentage (%) | | | Viral Antigen-Positive Cell Percentage (%) | | |
|---|---|---|---|---|---|---|---|---|---|
| | After 4 Days | After 7 Days | After 10 Days | After 4 Days | After 7 Days | After 10 Days | After 4 Days | After 7 Days | After 10 Days |
| 10 | 290 | 270 | 160 | 99 | 98 | 92 | ≤ 1 | 10 | 71 |
| 1 | 340 | 160 | 53 | 99 | 92 | 68 | 4 | 83 | 73 |
| 0.1 | 350 | 180 | 51 | 99 | 92 | 77 | 4 | 80 | 77 |
| 0 (control) | 300 | 50 | 36 | 95 | 59 | 51 | 86 | ≥90 | 85 |

Note: Cell count at the time of inoculation: $2.5 \times 10^5$ cells/ml. For explanation of terms, see Table 1.

Table 4

Efficacy of Ofloxacin on Cytophatic Effect in HIV-1-Infected CEM Cells

| Test Compound OFLX (μg/ml) | Viable Cell count ($x 10^4$ cells/ml) | | | | | Viable Cell Percentage (%) | | | | | Viral Antigen-Positive Cell Percentage (%) | | | | |
|---|---|---|---|---|---|---|---|---|---|---|---|---|---|---|---|
| | After 4 Days | After 7 Days | After 10 Days | After 20 Days | After 30 Days | After 4 Days | After 7 Days | After 10 Days | After 20 Days | After 30 Days | After 4 Days | After 7 Days | After 10 Days | After 20 Days | After 30 Days |
| 4-Day Addition: | | | | | | | | | | | | | | | |
| 10 | 260 | 63 | 280 | 310 | 320 | 94 | 73 | 85 | 92 | 95 | 74 | 63 | 40 | 14 | 3 |
| 5 | 250 | 60 | 17 | 320 | 330 | 93 | 59 | 77 | 86 | 97 | 82 | 50 | 33 | 5 | 4 |
| 2.5 | 260 | 58 | 83 | 300 | 300 | 96 | 67 | 67 | 95 | 97 | 80 | 67 | 49 | 4 | 6 |
| 1 | 60 | 43 | 13 | 34 | 10 | 96 | 53 | 21 | 87 | 37 | 88 | 8 | 77 | 16 | UN |
| Continuous Addition: | | | | | | | | | | | | | | | |
| 10 | 280 | 55 | 130 | 290 | 390 | 95 | 66 | 87 | 92 | 95 | 78 | 72 | 43 | 9 | 6 |
| 5 | 340 | 58 | 160 | 300 | 290 | 98 | 64 | 87 | 95 | 94 | 75 | 76 | 40 | 11 | 2 |
| 2.5 | 260 | 59 | 110 | 220 | 360 | 94 | 64 | 81 | 94 | 97 | 83 | 62 | 38 | 17 | 10 |
| 1 | 300 | 39 | 46 | 15 | 11 | 94 | 55 | 42 | 75 | 33 | 82 | 78 | 85 | UN | UN |
| 0 | 300 | 46 | 36 | 2 | 0 | 95 | 59 | 45 | 5 | 0 | 78 | ≥90 | 87 | UN | UN |

Note: UN: Undeterminable because of the presence of a lot of cell debris.
OFLX: Ofloxacin.
Cell count at the time of inoculation: $2.5 \times 10^5$ cells/ml.
For explanation of other terms, see Table 1.

Table 5

Efficacy of Fluorine-Containing Pyridonecarboxylic Acid Derivatives on Cytophatic Effect in HIV-1-Infected CEM Cells

| Test Compound (μg/ml) | Viable Cell count (x $10^4$ cells/ml) | | | | | Viable Cell Percentage (%) | | | | | Viral Antigen-Positive Cell Percentage (%) | | | | |
|---|---|---|---|---|---|---|---|---|---|---|---|---|---|---|---|
| | After 4 Days | After 7 Days | After 10 Days | After 20 Days | After 30 Days | After 4 Days | After 7 Days | After 10 Days | After 20 Days | After 30 Days | After 4 Days | After 7 Days | After 10 Days | After 20 Days | After 30 Days |
| IIIa 10 | 180 | 61 | 260 | 310 | 330 | 88 | 74 | 93 | 91 | 94 | 82 | 75 | 26 | 18 | 6 |
| 1 | 240 | 37 | 19 | 20 | 14 | 92 | 57 | 30 | 78 | 40 | 72 | 65 | 30 | 20 | UN |
| IIIb 10 | 190 | 62 | 240 | 320 | 300 | 89 | 70 | 83 | 92 | 94 | 80 | 50 | 40 | 13 | 4 |
| 1 | 220 | 53 | 19 | 25 | 14 | 89 | 75 | 27 | 81 | 36 | 88 | 62 | UN | UN | UN |
| IIIc 10 | 200 | 51 | 250 | 340 | 320 | 92 | 67 | 93 | 93 | 95 | 80 | 47 | 9 | 9 | 3 |
| 1 | 220 | 46 | 28 | 10 | 10 | 87 | 60 | 33 | 55 | 32 | 82 | 70 | UN | UN | UN |
| IIId 10 | 190 | 60 | 280 | 290 | 300 | 88 | 67 | 91 | 95 | 94 | 78 | 50 | 25 | 11 | 4 |
| 1 | 250 | 50 | 24 | 12 | 9 | 91 | 62 | 28 | 60 | 31 | 72 | 70 | UN | UN | UN |
| 0 (Control) | 270 | 47 | 20 | 7 | 0 | 9 | 55 | 31 | 9 | 0 | 81 | 80 | UN | UN | UN |

IIIa: (±)-9-Fluoro-3-methyl-10-(4-ethyl-1-piperazinyl)-7-oxo-2,3-dihydro-7H-pyrido[1,2,3-de][1,4]benzoxazine-6-carboxylic acid

IIIb: (±)-9-Fluoro-3-methyl-10-(4-n-propyl-1-piperazinyl)-7-oxo-2,3-dihydro-7H-pyrido[1,2,3-de][1,4]benzoxazine-6-carboxylic acid

IIIc: (±)-9-Fluoro-3-methyl-10-(4-n-butyl-1-piperazinyl)-7-oxo-2,3-dihydro-7H-pyrido[1,2,3-de][1,4]benzoxazine-6-carboxylic acid

IIId: (±)-9-Fluoro-3-methyl-10-(4-isopropyl-1-piperazinyl)-7-oxo-2,3-dihydro-7H-pyrido[1,2,3-de][1,4]benzoxazine-6-carboxylic acid

UN : Undeterminable because of the presence of a lot of cell debris.

Cell count at the time of inoculation: 2.5 x $10^5$ cells/ml.

For explanation of other terms, see Table 1.

Table 6

Efficacy of Levofloxacin on Cytophatic Effect in HIV-1-Infected CEM Cells

| Test Compound DR-3355 (μg/ml) | Viable Cell count ($\times 10^4$ cells/ml) | | | | | Viable Cell Percentage (%) | | | | | Viral Antigen-Positive Cell Percentage (%) | | | | |
|---|---|---|---|---|---|---|---|---|---|---|---|---|---|---|---|
| | After 4 Days | After 7 Days | After 10 Days | After 20 Days | After 30 Days | After 4 Days | After 7 Days | After 10 Days | After 20 Days | After 30 Days | After 4 Days | After 7 Days | After 10 Days | After 20 Days | After 30 Days |
| 4-Day Addition: | | | | | | | | | | | | | | | |
| 10 | 170 | 50 | 280 | 230 | 320 | 92 | 68 | 94 | 85 | 92 | 88 | 57 | 31 | 19 | ≤1 |
| 5 | 190 | 78 | 320 | 190 | 360 | 91 | 76 | 95 | 87 | 92 | 90 | 59 | 33 | 36 | 2 |
| 2.5 | 190 | 76 | 280 | 230 | 130 | 94 | 80 | 92 | 87 | 83 | 88 | 53 | 44 | 30 | 10 |
| 1 | 220 | 71 | 54 | 3 | 68 | 95 | 70 | 61 | 20 | 98 | 91 | 61 | 56 | UN | 16 |
| Continuous Addition: | | | | | | | | | | | | | | | |
| 10 | 160 | 43 | 220 | 260 | 260 | 91 | 75 | 92 | 87 | 83 | 89 | 55 | 31 | 21 | 4 |
| 5 | 160 | 51 | 260 | 320 | 210 | 89 | 66 | 95 | 89 | 75 | 87 | 53 | 37 | 29 | 4 |
| 2.5 | 210 | 53 | 290 | 390 | 270 | 94 | 68 | 94 | 95 | 88 | 87 | 51 | 25 | 35 | 11 |
| 1 | 190 | 65 | 110 | 11 | 360 | 94 | 69 | 61 | 50 | 98 | 88 | 52 | 43 | UN | 15 |
| 0 (Control) | 180 | 48 | 27 | 4 | 0 | 92 | 66 | 38 | 4 | 0 | 89 | 75 | UN | UN | UN |

UN: Undeterminable because of the presence of a lot of cell debris.
DR-3355: Levofloxacin.
Cell count at the time of inoculation: $2.5 \times 10^5$ cells/ml.
For explanation of other terms, see Table 1.

Table 7

Efficacy of Norfloxacin on Cytophatic Effect in HIV-1-Infected CEM Cells

| Test Compound NFLX (μg/ml) | Viable Cell count ($x 10^4$ cells/ml) | | | | | Viable Cell Percentage (%) | | | | | Viral Antigen-Positive Cell Percentage (%) | | | | |
|---|---|---|---|---|---|---|---|---|---|---|---|---|---|---|---|
| | After 4 Days | After 7 Days | After 10 Days | After 20 Days | After 30 Days | After 4 Days | After 7 Days | After 10 Days | After 20 Days | After 30 Days | After 4 Days | After 7 Days | After 10 Days | After 20 Days | After 30 Days |
| 10 | 180 | 60 | 250 | 370 | 400 | 92 | 71 | 91 | 97 | 94 | 92 | 63 | 50 | 4 | ≤1 |
| 5 | 230 | 84 | 86 | 28 | 330 | 94 | 67 | 61 | 81 | 95 | 83 | 65 | UN | UN | ≤1 |
| 2.5 | 240 | 59 | 40 | 2 | 70 | 94 | 67 | 47 | 33 | 96 | 93 | 55 | UN | UN | ≤1 |
| 1 | 230 | 52 | 26 | 0 | 0 | 94 | 59 | 30 | 0 | 0 | 88 | 75 | UN | UN | NT |
| 0 | 188 | 52 | 32 | 1 | 0 | 95 | 58 | 43 | 14 | 0 | 87 | 78 | UN | UN | NT |

NFLX: Norfloxacin.
UN : Undeterminable because of the presence of a lot of cell debris.
NT : Not tested.
Cell count at the time of inoculation: $2.5 x 10^5$ cells/ml.
For explanation of other terms, see Table 1.

Table 8

Efficacy of Coprofloxacin and Enoxacin on Cytophatic Effect in HIV-1-Infected CEM Cells

| Test Compound (μg/ml) | Viable Cell count (x $10^4$ cells/ml) | | | | | Viable Cell Percentage (%) | | | | | Viral Antigen-Positive Cell Percentage (%) | | | | |
|---|---|---|---|---|---|---|---|---|---|---|---|---|---|---|---|
| | After 4 Days | After 7 Days | After 10 Days | After 20 Days | After 30 Days | After 4 Days | After 7 Days | After 10 Days | After 20 Days | After 30 Days | After 4 Days | After 7 Days | After 10 Days | After 20 Days | After 30 Days |
| CPFX | | | | | | | | | | | | | | | |
| 10 | 180 | 65 | 250 | 300 | 330 | 91 | 77 | 92 | 96 | 95 | 88 | 57 | 27 | 10 | ≤1 |
| 5 | 220 | 76 | 310 | 280 | 320 | 97 | 75 | 92 | 95 | 92 | 92 | 67 | 29 | 4 | ≤1 |
| 2.5 | 200 | 98 | 320 | 220 | 360 | 95 | 77 | 85 | 95 | 97 | 87 | 60 | 31 | 3 | ≤1 |
| 1 | 220 | 86 | 52 | 8 | 33 | 84 | 73 | 53 | 53 | 92 | 83 | 62 | 37 | 33 | NT |
| ENX | | | | | | | | | | | | | | | |
| 10 | 140 | 52 | 150 | 178 | 379 | 97 | 61 | 91 | 96 | 94 | 88 | 68 | 30 | 5 | ≤1 |
| 5 | 210 | 58 | 78 | 11 | 310 | 98 | 60 | 62 | 11 | 98 | 93 | 68 | UN | UN | ≤1 |
| 2.5 | 240 | 67 | 30 | 1 | 2 | 95 | 68 | 35 | 13 | 67 | 90 | 65 | UN | UN | NT |
| 1 | 220 | 64 | 29 | 7 | 0 | 95 | 66 | 41 | 50 | 0 | 88 | 70 | UN | UN | NT |
| 0 | 194 | 66 | 32 | 1 | 0 | 93 | 68 | 42 | 17 | 0 | 88 | 73 | UN | UN | NT |

CPFX: Ciprofloxacin hydrochloride.
ENX : Enoxacin.
UN : Undeterminable because of the presence of a lot of cell debris.
NT : Not tested.
Cell count at the time of inoculation: 2.5 x $10^5$ cells/ml.
For explanation of other terms, see Table 1.

Table 9

Efficacy of Combined Use of Azidothymidine and Levofloxacin on Cytophatic Effect in HIV-1-Infected CEM Cells

| Test Compound (µg/ml) | | Viable Cell Count ($x\ 10^4$ cells/ml) | | | | | | Viable Cell Percentage (%) | | | | | | Viral Antigen-Positive Cell Percentage (%) | | | | | |
|---|---|---|---|---|---|---|---|---|---|---|---|---|---|---|---|---|---|---|---|
| | | Number of Days After Viral Infection | | | | | | | | | | | | | | | | | |
| AZT* | DR-3355** | 4 | 7 | 10 | 15 | 20 | 30 | 4 | 7 | 10 | 15 | 20 | 30 | 4 | 7 | 10 | 15 | 20 | 30 |
| 4-Day Addition: | | | | | | | | | | | | | | | | | | | |
| 0 | 0 | 290 | 45 | 19 | 3 | 0 | 0 | 98 | 51 | 21 | 15 | 0 | 0 | 50 | 87 | 89 | 60 | NT | NT |
| 0 | 1 | 300 | 59 | 23 | 27 | 220 | 260 | 98 | 58 | 30 | 54 | 96 | 97 | 51 | 81 | 83 | 33 | 9 | 1 |
| 0 | 10 | 250 | 71 | 240 | 310 | 340 | 310 | 98 | 63 | 81 | 95 | 95 | 95 | 49 | 81 | 29 | 16 | 11 | 1 |
| 0.1 | 0 | 320 | 230 | 200 | 8 | 0 | 0 | 98 | 96 | 75 | 24 | 0 | 0 | 4 | 73 | 84 | 65 | NT | NT |
| 0.1 | 1 | 350 | 250 | 200 | 30 | 110 | 260 | 99 | 95 | 71 | 41 | 97 | 95 | 4 | 67 | 74 | 62 | 20 | 1 |
| 0.1 | 10 | 260 | 200 | 160 | 170 | 280 | 290 | 98 | 95 | 59 | 83 | 96 | 94 | 2 | 59 | 69 | 27 | 12 | 1 |
| 1 | 0 | 280 | 220 | 240 | 9 | 0 | 0 | 98 | 98 | 91 | 17 | 0 | 0 | <1 | 9 | 64 | 74 | NT | NT |
| 1 | 1 | 360 | 270 | 290 | 30 | 87 | 320 | 98 | 97 | 90 | 43 | 92 | 97 | <1 | 22 | 76 | 35 | 25 | 2 |
| 1 | 10 | 250 | 230 | 220 | 94 | 310 | 320 | 96 | 96 | 74 | 71 | 96 | 95 | <1 | 34 | 41 | 40 | 17 | 2 |
| 0 | 0 | 260 | 53 | 54 | 4 | 0 | 0 | 95 | 59 | 56 | 17 | 0 | 0 | 91 | 70 | UN | UN | NT | NT |
| Continous Addition: | | | | | | | | | | | | | | | | | | | |
| 0 | 1 | 310 | 100 | 84 | 200 | 300 | 270 | 96 | 74 | 62 | 89 | 97 | 92 | 92 | 53 | 33 | UN | 9 | <1 |
| 0 | 10 | 240 | 80 | 240 | 260 | 300 | 200 | 96 | 78 | 88 | 94 | 94 | 90 | 90 | 58 | 28 | 23 | 4 | <1 |
| 0.1 | 0 | 380 | 230 | 150 | 8 | 1 | 0 | 99 | 88 | 70 | 19 | 7 | 0 | 16 | 87 | 70 | 88 | UN | NT |
| 0.1 | 1 | 350 | 220 | 190 | 69 | 250 | 210 | 99 | 89 | 67 | 72 | 92 | 90 | 9 | 83 | 85 | 75 | 13 | <1 |
| 0.1 | 10 | 280 | 170 | 160 | 250 | 290 | 270 | 99 | 87 | 66 | 89 | 94 | 91 | 10 | 95 | 57 | 50 | 13 | <1 |

Note: AZT*: Azidothymidine
DR-3355**: Levofloxacin

## Claims

1. An anti-human immunodeficiency virus (HIV) agent which comprises a fluorine-containing pyridonecarboxylic acid derivative as an active ingredient.

2. An anti-HIV agent as claimed in Claim 1, wherein said fluorine-containing pyridonecarboxylic acid derivative is a 1-cyclopropyl (or 1-halo-substituted-cyclopropyl)-6-fluoro-1,4-dihydro-4-oxo-7-nitrogen-containing heterocycle-substituted-3-quinolinecarboxylic acid, which may optionally have a halogen atom, a lower alkyl group or a lower alkoxy group as a substituent at 8-position.

3. An anti-HIV agnet as claimed in Claim 1, wherein said fluorine-containing pyridonecarboxylic acid derivative is a 1-lower alkyl-6-fluoro-1,4-dihydro-4-oxo-7-(1-piperazinyl- or lower alkyl-substituted 1-piperazin-yl)-1,8-naphthyridine-3-carboxylic acid.

4. An anti-HIV agent as claimed in Claim 1, wherein said fluorine-containing pyridonecarboxylic acid derivative is an 1-ethyl-6-fluoro-1,4-dihydro-4-oxo-7-(1-piperazinyl or lower alkyl-substituted 1-piperazinyl)-3-quinolinecarboxylic acid, which may optionally have a halogen atom, a lower alkyl group or a lower alkoxy group as a substituent at 8-position.

5. An anti-HIV agent as claimed in Claim 1, wherein said fluorine-containing pyridonecarboxylic acid derivative is a 9-fluoro-2,3-dihydro-3-methyl-10-(4-lower alkyl-1-piperazinyl)-7-oxo-7H-pyrido-[1,2,3-de]-[1,4]benzoxazine-6-carboxylic acid, a salt thereof or a hydrate thereof.

6. An anti-HIV agent as claimed in Claim 1, wherein said fluorine-containing pyridonecarboxylic acid derivative is a 9-fluoro-2,3-dihydro-3-methyl-10-(4-lower alkyl-1-piperazinyl)-7-oxo-7H-pyrido[1,2,3-de]-[1,4]benzoxazine-6-carboxylic acid in 3S form.

7. An anti-HIV agent as claimed in Claim 1, wherein said fluorine-containing pyridonecarboxylic acid derivative is ofloxacin, the 3S form thereof (levofloxacin), ciprofloxacin, enoxacin, norfloxacin, lomefloxacin, fleroxacin, difloxacin, tosufloxacin (T-3262), A-62254, AT-3765 or AM-1091.

8. A pharmaceutical composition for enhanced anti-HIV effect, which comprises a fluorine-containing pyridonecarboxylic acid derivative as an active ingredient in combination with azidothymidine, dideoxyinosine or dideoxycytidine.

9. A pharmaceutical composition as claimed in Claim 8, wherein said fluorine-containing pyridonecarboxylic acid derivative is a 1-cyclopropyl (or 1-halo-substituted-cyclopropyl)-6-fluoro-1,4-dihydro-4-oxo-7-nitrogen-containing heterocycle-substituted-3-quinolinecarboxylic acid, which may optionally have a halogen atom, a lower alkyl group or a lower alkoxy group as a substituent at 8-position.

10. A pharmaceutical composition as claimed in Claim 8, wherein said fluorine-containing pyridonecarboxylic acid derivative is a 1-lower alkyl-6-fluoro-1,4-dihydro-4-oxo-7-(1-piperazinyl- or lower alkyl-substituted 1-piperazinyl)-1,8-naphthyridine-3-carboxylic acid.

11. A pharmaceutical composition as claimed in Claim 8, wherein said fluorine-containing pyridonecarboxylic acid derivative is an 1-ethyl-6-fluoro-1,4-dihydro-4-oxo-7-(1-piperazinyl or lower alkyl-substituted 1-piperazinyl)-3-quinolinecarboxylic acid, which may optionally have a halogen atom, a lower alkyl group or a lower alkoxy group as a substituent at 8-position.

12. A pharmaceutical composition as claimed in Claim 8, wherein said fluorine-containing pyridonecarboxylic acid derivative is a 9-fluoro-2,3-dihydro-3-methyl-10-(4-lower alkyl-1-piperazinyl)-7-oxo-7H-pyrido-[1,2,3-de][1,4]benzoxazine-6-carboxylic acid, a salt thereof or a hydrate thereof.

13. A pharmaceutical composition as claimed in Claim 8, wherein said fluorine-containing pyridonecarboxylic acid derivative is ofloxacin, the 3S form thereof (levofloxacin), ciprofloxacin, enoxacin, norfloxacin, lomefloxacin, fleroxacin, difloxacin, tosufloxacin (T-3262), A-62254, AT-3765 or AM-1091.

14. An anti-HIV agent which comprises a fluorine-containing pyridonecarboxylic acid derivative and azidothymidine, deoxycytidine or deoxyinosine as active ingredients.

# INTERNATIONAL SEARCH REPORT

International Application No PCT/JP90/00571

**I. CLASSIFICATION OF SUBJECT MATTER** (if several classification symbols apply, indicate all) [6]

According to International Patent Classification (IPC) or to both National Classification and IPC Int. Cl[5]

C07D215/56,C07D471/04,C07D489/06,C07D513/06,A61K31/435,A61K31/47, A61K31/495,A61K31/535,A61K31/54

**II. FIELDS SEARCHED**

Minimum Documentation Searched [7]

| Classification System | Classification Symbols |
|---|---|
| IPC | C07D215/56, C07D471/04, C07D489/06, C07D513/06, A61K31/435, A61K31/47, A61K31/495, A61K31/535, A61K31/54 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| Y | JP, A, 63-145268 (Bayer A.G.), 17 June 1988 (17. 06. 88) & EP, A, 274033 & DE, A, 3641312 | 1-4, 7-11, 13, 14 |
| X,Y | JP, A, 60-258163 (Bayer A.G.), 20 December 1985 (20. 12. 85) & US, A, 4659603 & EP, A, 165474 & DE, A, 3420116 | 1, 2, 4, 7-9, 11, 13 14 |
| X,Y | JP, A, 60-202822 (Daiichi Seiyaku Co., Ltd.), 14 October 1985 (14. 10. 85), (Family: none) | 1, 5-8, 12-14 |
| A | JP, A, 62-19587 (Rhône-Poulene Santé), 28 January 1987 (28. 01. 87) & US, A, 4720500 & EP, A, 208621 & FR, A, 2584722 | 1, 3, 7, 8, 10, 13, 14 |
| A | JP, A, 50-18480 (CIBA-Geigy A.G.), 26 February 1975 (26. 02. 75) & US, A, 3960868 & DE, A, 2421121 & FR, A, 2228488 | 1, 2, 4, 7-9, 11, 13 14 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| July 5, 1990 (05. 07. 90) | July 23, 1990 (23. 07. 90) |
| **International Searching Authority** | **Signature of Authorized Officer** |
| Japanese Patent Office | |